# EUROPEAN PATENT APPLICATION

(11) **EP 4 759 309 A2**
(43) Date of publication of application: **17.06.2026**
(21) Application number: 25224667.3
(22) Date of filing: 17.12.2025
(51) Int. Cl.: A61K 31/197, A61K 9/00, A61K 31/198, A61K 31/426, A61K 31/454, A61P 31/10

(54) **KYNURENINE PATHWAY ENZYME INHIBITORS FOR USE IN THE TREATMENT OF MYCOSES CAUSED BY TRICOPHYTON RUBRUM**

(30) Priority: 16.12.2024 PL 45060524
(71) Applicant: Uniwersytet Lodzki, 90-136 Lodz (PL); Katolicki Uniwersytet Lubelski Jana Pawla II, 20-059 Lublin (PL); Uniwersytet Medyczny w Lublinie, 20-059 Lublin (PL)
(72) Inventor: Ciesielska, Anita, 95-030 Starowa Gora (PL); Dzitko, Katarzyna, 95-010 Dobieszkow (PL); Straczek, Pawel, 95-010 Swedow (PL); Turski, Waldemar, 20-868 Lublin (PL); Sobczynski, Jan, 20-802 Lublin (PL); Turska-Kozlowska, Monika, 20-882 Lublin (PL)
(74) Representative: Kicinska-Fujawa, Alicja

(57) **Abstract**

The object of the invention is an inhibitor of the kynurenine pathway enzyme selected from a group comprising 1-methyltryptophan, aminooxyacetic acid, 2-aminoadipic acid, 3,4-dimethoxy-N-[4-(3-nitrophenyl)-2-thiazolyl]-benzenesulfonamide) and (2-(3,4 dimethoxybensenosulfonylamin)-4-(3-nitrophenyl)-5-(piperidin-1-yl)methylthiazole) for use as a drug used in the prevention or treatment of fungal infections, especially those caused by *Trichophyton rubrum,* intended for topical administration.

## Description

The subject of the invention is an inhibitor of the kynurenine pathway enzyme as a *Trichophyton rubrum* growth regulator for use in the prevention or treatment of fungal infections, especially those caused by *Trichophyton rubrum.*

*Trichophyton rubrum (T. rubrum)* is the most common anthropophilic dermatophyte found on human skin, responsible for over 60% of all superficial mycoses in humans. It causes fungal infections of the nails and feet, as well as the smooth skin and groin. Unlike other fungi, dermatophytes can attack healthy people with normally functioning immune system. The fungus is transmitted through human-to-human contact with a carrying person, and infection with this dermatophyte is a worldwide problem.

Topical and/or systemic drugs are currently used in dermatophytoses. Topical medications recommended and most commonly used for nail fungal infections include ciclopirox and amorolfine in nail polish form. Imidazole derivatives in the form of creams, solutions and gels (e.g. clotrimazole, ketoconazole), allylamines in the form of creams (e.g. terbinafine), amorolfine in cream and ciclopirox in the form of cream, gel or suspension are used in foot, groin and smooth skin mycoses. Itraconazole, fluconazole and oral terbinafine are used for general treatment in all of the foregoing dermatophytoses. Using medications in a pulsed (cyclic) method is recommended [1].

Despite the wide availability of antifungal medications, treating superficial mycoses remains a challenge due to the need for systematic, long-term therapy, the location of lesions in poorly vascularised areas, which significantly hinders drug penetration and its efficacy, as well as developing tolerance and resistance of dermatophytes to available drugs [2].

Combined treatment is recommended in patients with extensive mycoses, in the elderly and in those for whom previous treatment has not been successful. Combined treatment may involve using two different systemic drugs, in separate pulses following sequentially, or using systemically administered drugs with the concomitant use of topically acting drugs [3].

Tryptophan is a building block amino acid for living organisms. For humans, tryptophan is an essential amino acid that is supplied to the body with food. In contrast, fungi, including *T. rubrum* produce tryptophan exogenously [4]. In addition to its building function in protein synthesis, tryptophan undergoes numerous metabolic transformations in the indole, serotonin and kynurenine pathways [5]. Tryptophan metabolism in the dermatophyte *T. rubrum* is unknown.

The term "kynurenine pathway" is used to describe tryptophan's metabolic cycle. The pathway starts with the conversion of tryptophan to kynurenine. The main pathway leads to the formation of quinolinic acid, which is the precursor of nicotinamide adenine dinucleotide (NAD) through subsequent enzymatic transformations. Tryptophan is converted to kynurenine under the influence of the enzymes: tryptophan 2,3-dioxygenase (TDO) (EC 1.13.11.11) and indoleamine 2,3-dioxygenase (IDO) (EC 1.13.11.52). Kynurenine is converted by kynurenine 3-monooxygenase (KMO) (EC 1.14.13.9) to 3-hydroxykynurenine. 3-Hydroxykynurenine is converted to 3-hydroxyanthranilic acid under the influence of kynureninase (EC 3.7.1.3). Under the influence of 3,4-hydroxyanthranilate dioxygenase (EC 1.13.11.6), an intermediate compound called 2-amino-3-carboxymuconium semialdehyde is formed from it, which spontaneously converts to quinolinic acid. The side branches of the kynurenine pathway also produce anthranilic acid under the influence of kynureninase, and kynurenic acid and xanthurenic acid under the influence of kynurenine aminotransferase, also referred to as KAT in the following description, occurring in four isoforms. In addition, cinnabaric acid and spontaneously picolinic acid are formed as a result of autooxidation. The processes described have been defined in animals [6].

The enzymatic processes involved in the kynurenine pathway are inhibited by the inhibitors: 1-methyltryptophan [ 1-methyl-D-tryptophan, indoximod, molecular formula C₁₂H₁₄N₂O₂, CAS 110117-83-4]; aminooxyacetic acid [molecular formula C₂H₅NO₃, CAS No. 645-88-5]; 2-aminoadipic acid [molecular formula C₆H₁₁NO₄, CAS No. 542-32-5], the inhibitor Ro 61-8048 [3,4-dimethoxy-N-[4-(3-nitrophenyl)-2-thiazolyl] benzenesulfonamide, molecular formula C₁₇H₁₅N₃O₆S₂, CAS No. 199666-03-0] and KMO Inhibitor II [JM6, 3,4-dimethoxy-N-(4-(3-nitrophenyl)-5-(piperidin-1-ylmethyl)thiazol-2-yl) benzenesulfonamide, molecular formula C₂₃H₂₆N₄O₆S₂, CAS number 1008119-83-2].

US patent application US2024234623 discloses a method of treating neoplastic disorders or infections by administering an indoleamine-2,3-dioxygenase inhibitor, such as 1-methyltryptophan, to reverse IDO-dependent immunosuppression, in combination with another therapeutic agent (e.g. chemotherapy or radiotherapy).

The tryptophan conversion to kynurenine is catalysed by 3 enzymes: TDO, IDO1 and IDO2.

1-Methyltryptophan is an inhibitor of both IDO1 and IDO2 enzymes. It binds to the enzyme protein but is not metabolised, inactivating the enzyme instead. It is believed that IDO2 may also have distinct, non-enzymatic biological activity [7]. 1-Methyltryptophan is tested in clinical trials as an adjunctive component in combination therapy for various neoplastic disorders [8].

Aminooxyacetic acid is a non-selective aminotransferase inhibitor [9]. It inhibits KAT activity [10].

Chinese patent application No. CN10984687 discloses the use of aminooxyacetic acid in the treatment of hepatocellular carcinoma, indicating its ability to reduce the O-N-acetylglucosamine modification level in neoplastic cells and inhibit their proliferation, invasion and metastasis.

2-Aminoadipic acid inhibits kynurenic acid synthesis by affecting KAT II [11, 12]. 2-Aminoadipic acid is a substrate in the lysine production pathway in pathogenic fungi, and enzyme inhibitors of the so-called aminoadipic pathway are considered to be antifungal medications [13]. This makes it all the more surprising that this substance shows antifungal activity.

The international application WO2014164404 describes the use of 2-aminoadipic acid for the treatment of metabolic disorders related to glucose metabolism.

Greco et al. [14] described the uses of 3,4-dimethoxy-N-[4-(3-nitrophenyl)-2-thiazolyl]-benzenesulfonamide (Ro 61-8048, Fig. 12) and its analogue 2-(3,4-dimethoxybenzenesulfonylamino)-4-(3-nitrophenyl)-5-(piperidin-1-yl)methylthiazole (JM6, Fig. 13) as KMO inhibitors for the treatment of disorders dependent on this enzyme activity, including neurodegenerative, inflammatory and metabolic disorders.

Kynurenine aminotransferase (KAT) is an enzyme that catalyses the conversion of kynurenine to kynurenic acid and 3-hydroxykynurenine to xanthurenic acid. Four isoforms of the enzyme have been defined in the human body and named with consecutive Roman numbers: KAT I (i.e. glutamine transaminase K/cysteine conjugate beta-lyase 1), KAT II (i.e. aminoadipate aminotransferase), KAT III (i.e. cysteine conjugate beta-lyase 2) and KAT IV (i.e. glutamic-oxaloacetic transaminase 2/mitochondrial aspartate aminotransferase) [15]. Because it catalyses the kynurenic acid formation, this enzyme has attracted interest as a potential therapeutic target in central nervous system disorders [16]. In 2019, the initiation of a Phase I and Phase II clinical trial with this enzyme inhibitor called N-acetylcysteine (clinical trial identifier NCT04013555) was announced to assess its effects on central nervous system function.

In addition to the above-mentioned substances, a number of KAT inhibitors have been described [16].

The chemical structure of kynurenine aminotransferase (KAT) inhibitors is disclosed in the international description of the invention WO2009064836A.

Kynurenine 3-monooxygenase is an enzyme associated with the outer mitochondrial membrane in eukaryotic cells. The enzyme regulates the activity of the main tryptophan metabolism pathway, responsible for approximately 95% of this amino acid metabolism. Because of that, it is attracting interest as a potential therapeutic target in neurodegenerative disorders, psychiatric disorders and neoplastic disorders [17]. In 2017, a phase I clinical trial with this enzyme inhibitor called GSK3335065 (clinical trial identifier NCT03245619) was started, and terminated due to adverse events. The studied substance was intended to have applications in the acute pancreatitis treatment. In addition to the aforementioned substance, kynurenine 3-monooxygenase inhibitors include m-NBA (i.e. m-nitrobenzoyl)-alanine), UPF 648 (i.e.: (15,25)-2-(3,4-dichlorobenzoyl)cyclopropanecarboxylic acid) and Ro-61-8048 (3,4-dimethoxy-[-N-4-(nitrophenyl)thiazol-2yl]-benzenesulfonamide), and FCE 28833A (3,4-dichlorobenzoyl alanine), which are kynurenine analogues. Allosteric inhibition of the enzyme is induced by sulphonamide derivatives Ro 61-8048, KMO Inhibitor II (JM6), N-(6-phenylpyridazin-3-yl) benzenesulphonamide, N-(6-(5-fluoro-2-(piperidin-1-yl) phenyl)pyridazin-3-yl)-1-(tetrahydro-2H-pyran-4-yl) methanesulphonamide, P323-0389, F6548-0495. Other inhibitors include GSK065, GSK180, GSK366, GSK428, GSK775, GSK891, GSK3335065, lanthellamide A, diclofenac, ZINC-71915355, ZINC-19827377, CHDI-340246 [18, 19].

The chemical structure of the kynurenine 3-monooxygenase inhibitors is disclosed in the descriptions of the international invention applications under application Nos. WO1997017317A1, EP1424333A1, EP1475385A1, WO2008022281A1, WO2008022286A2, WO2010011302A1, WO2010017179A1, WO2010017132A1, WO2011091153A1, WO2013016488A1, WO2013033085A1, WO2013151707A1, WO2015091647A1.

There is no information in the available literature regarding the use of kynurenine pathway enzyme inhibitors as a growth regulator of *Trichophyton rubrum* for the prevention or treatment of mycoses, especially those caused by *Trichophyton rubrum.*

The technical problem posed by the present invention is to provide prevention and treatment of mycoses, specifically caused by *Trichophyton rubrum.*

The subject of the invention is an inhibitor of the kynurenine pathway enzyme selected from the group comprising 1-methyltryptophan, aminooxyacetic acid, 2-aminoadipic acid, 3,4-dimethoxy-N-[4-(3-nitrophenyl)-2-thiazolyl]-benzenesulfonamide) and (2-(3,4-dimethoxybensenosulfonylamino)-4-(3-nitrophenyl)-5-(piperidin-1-yl)methylthiazole) alone or in combination for use in the prevention or treatment of mycoses, especially those caused by *Trichophyton rubrum.*

The terms "treat" or "treatment" refer to the way in which the disorder and/or its accompanying symptoms are relieved or remedied. The terms "prevent" or "prevention" refer to a method of reducing the likelihood or eliminating the possibility of developing a disorder.

As shown in the embodiments, inhibitors of the kynurenine pathway enzyme have varying levels of growth-inhibiting concentrations for the fungus *Trichophyton rubrum.* In the case of aminooxyacetic acid, its inhibitory effect on fungal growth was already achieved at a concentration of 1.25 mM, while in the case of 1-methyltryptophan, a concentration of 5 mM is needed to achieve this effect. In the case of 2-aminoadipic acid, 3,4-dimethoxy-N-[4-(3-nitrophenyl)-2-thiazolyl]-benzenesulphonamide and 2-(3,4 dimethoxybensenosulfonylamino)-4-(3-nitrophenyl)-5-(piperidin-1-yl)methylthiazole, the fungus growth inhibitory concentration is 2.5 mM.

Therefore, inhibitors of the kynurenine pathway enzyme for use according to the invention selected from the group comprising 1-methyltryptophan, aminooxyacetic acid, 2-aminoadipic acid, 3,4-dimethoxy-N-[4-(3-nitrophenyl)-2-thiazolyl]-benzenesulfonamide) and (2-(3,4-dimethoxybensenosulfonylamino)-4-(3-nitrophenyl)-5-(piperidin-1-yl)methylthiazole) or combinations thereof should be used at a minimum effective concentration, the minimum effective concentration being, respectively: 1.25 mM for aminooxyacetic acid; 2.5 mM for 2-aminoadipic acid; 3,4-dimethoxy-N-[4-(3-nitrophenyl)-2-thiazolyl]-benzenesulfonamide, 2-(3,4 dimethoxybensenosulfonylamino)-4-(3-nitrophenyl)-5-(piperidin-1-yl)methylthiazole , and 5 mM for 1-methyltryptophan.

The kynurenine pathway enzyme inhibitor for use according to the invention can be formulated into a variety of pharmaceutical forms, such as dust, nail polish, powder, by known formulation methods, including additives including excipients, film-forming agents, solubilising agents and other pharmaceutically acceptable additives.

Since *Trichophyton rubrum* causes mycoses of the fingernails and toenails, the inhibitor for use according to the invention is administered topically preferably in the form of a polish that can be applied to the affected nails.

According to the invention, the composition of a medicinal nail polish containing the inhibitor comprises a base in which an active substance with antifungal activity is dissolved or suspended. The penetration of an active substance is inversely proportional to its molecular weight and also depends on the ionisation of the active substance, the wetting angle of the preparation, the adhesion of the liquid on the nail plate surface and the degree of fineness of the active substance that is suspended in the base. Therefore, the polish should exhibit adhesion to allow prolonged activity on the nail surface and easy wettability resulting in good plate coverage. In addition, the polish composition should have low viscosity, low surface tension.

In the field of therapeutic nail formulations, the prior art describes anhydrous, hydrophilic and oil-based nail polish compositions.

The anhydrous nail polishes preferably contain: organic solvents such as ethyl acetate, butyl acetate, anhydrous ethyl alcohol, isopropyl alcohol, triacetin, thickening ingredients, e.g. cetostearyl alcohol, and components forming film on the nail surface, such as methyl vinyl ether copolymer with monobutyl maleate (1:1), ammonium methacrylate copolymer (type A), methoxyethene polymer with 2-butenedioic acid monobutyl ester, etc.

On the other hand, hydrophilic nail polishes preferably contain: polar solvents such as water, propylene glycol, ethyl alcohol; viscosity enhancers: hydroxypropyl cellulose, hydroxypropyl methylcellulose; nail plate penetration enhancers: carbocysteine, N-acetylcysteine, lactic acid, polyoxoethylene glycol 300 or 400, sodium dodecyl sulphate, polyethyleneglycol 6, oleyl or linoleyl ether, polyethyleneglycol 8, caprylic and caproic acids glyceride; wetting agents: cyclomethicone, dimethicone; surface tension reducing agents such as myristyl lactate, diisopropyl adipate, solubilisers such as Polyoxyl 40 hydrogenated castor oil, beta-cyclodextrin, hydroxybeta-cyclodextrin; preservatives; pH modifiers such as sodium hydroxide, hydrochloric acid.

The formulation of an anhydrous nail polish according to the invention can be either an anhydrous polish with exemplary compositions shown in Table 1, or a hydrophilic polish with compositions shown in Table 2, the active ingredient in each variant composition being any of the following inhibitors of the kynurenine pathway enzymes:
- 1-Methyltryptophan (English name: 1-methyl-D-tryptophan, indoximod) has the total molecular formula C₁₂H₁₄N₂O₂; CAS number 110117-83-4;
- Aminooxyacetic acid has the total molecular formula C₂H₅NO₃; CAS number 645-88-5;
- 2-aminoadipic acid has the total molecular formula C₆H₁₁NO₄; CAS number 542-32-5;
- Ro 61-8048 (3,4-dimethoxy-N-[4-(3-nitrophenyl)-2-thiazolyl]-benzenesulfonamide) has the total molecular formula C₁₇H₁₅N₃O₆S₂; CAS number 199666-03-0;
- KMO Inhibitor II (JM6) (3,4-dimethoxy-N-(4-(3-nitrophenyl)-5-(piperidin-1-ylmethyl)thiazol-2-yl)benzenesulfonamide) has the total molecular formula C₂₃H₂₆N₄O₆S₂; CAS number 1008119-83-2.

**Table 1 Waterless nail polish compositions**

| **Substance** | **CAS No** | **Concentration [wt.%]** |
|---|---|---|
| Active substance | | 1-5 |
| Ethyl alcohol | 64-17-5 | 40-60 |
| Butyl acetate | 123-86-4 | 5-25 |
| Ethyl acetate | 141-78-6 | 20-40 |
| Triacetin | 102-76-1 | 1-5 |
| Ammonium methacrylate copolymer (Type A) | 33434-24-1 | 0.5-2.5 |

**Table 2 Hydrophilic polish composition**

| **Substance** | **CAS No** | **Concentratio n [wt.%]** |
|---|---|---|
| Active substance | | 1-5 |
| Polyoxyl 40 hydrogenated castor oil | 61788-85-0 | 1-10 |
| Ethyl alcohol | 64-17-5 | 10-30 |
| Propylene glycol | 57-55-6 | 0.5-2.0 |
| Low viscosity Hydroxypropyl methylcellulose | 9004-65-3 | 0.1-1.0 |
| Dimethicone | 9006-65-9 | 0.1-1.0 |
| Hydrochloric acid | 7647-01-0 | 0.01-0.1 |
| Purified water | 7732-18-5 | 20-80 |

Experiments carried out by the inventors of the invention have shown that kynurenine pathway enzyme inhibitors selected from the group comprising 1-methyltryptophan, aminooxyacetic acid, 2-aminoadipic acid, Ro 61-8048 and KMO Inhibitor II (JM6) inhibit or limit the growth of the fungus *Trichophyton rubrum,* and can therefore be used in the prevention or treatment of infection caused by this fungus.

Since it is common practice in the topical treatment of fungal infections, especially those resistant to treatment, to use compositions containing combinations of different antifungal agents in order to increase their effectiveness, inhibitors of enzymes of the kynurenine pathway selected from a group including 1-methyltryptophan, aminooxyacetic acid, 2-aminoadipic acid, Ro 61-8048 and KMO Inhibitor II (JM6) can also be used in compound compositions for external application in combination with other antifungal agents.

For particularly resistant mycoses, it is medical practice to use topically acting drugs and systemically acting drugs, therefore kynurenine pathway enzyme inhibitors selected from a group comprising 1-methyltryptophan, aminooxyacetic acid, 2-aminoadipic acid, Ro 61-8048 and KMO Inhibitor II (JM6) can be used in combination treatment, involving a combination of topically acting drugs, such as nail polish, and systemically acting drugs, such as a tablet.

As antifungal agents acting on *Trichophyton rubrum* are also effective against dermatoses caused by other fungi, given the similarity of fungal metabolism and the clinical data available in the literature, kynurenine pathway enzyme inhibitors selected from a group including 1-methyltryptophan, aminooxyacetic acid, 2-aminoadipic acid, Ro 61-8048 and KMO Inhibitor II (JM6) can be used to prevent infection and control diseases caused by other fungi.

In the detailed example of the invention presented below, a description of the experimental work carried out by the inventors is provided, whereby the results presented do not limit the invention.

### EXAMPLE 1

### Evaluation of antifungal activity of kynurenine pathway enzyme inhibitors

Testing the antifungal activity of kynurenine pathway enzyme inhibitors was carried out on a reference strain of *Trichophyton rubrum,* from the Dutch collection of the Westerdijk Fungal Biodiversity Institute, deposited under No. CBS 120358.

The microbial activity of kynurenine pathway enzyme inhibitors was tested by determining the Minimum Inhibitory Concentration (MIC), by serial dilutions in a 96-well plate, based on the EUCAST standard [20].

*Trichophyton rubrum* CBS 120358 cultures (14 - 21 days old) on slants with solid Sabouraud medium were washed with 5 mL of a 0.1% solution of sterile distilled water with Tween 20. The mycelium was then harvested with a sterile wire loop and aspirated through a filter (Corning Sterile Cell Strainer, 40 µm) into a 50 mL Falcon tube and centrifuged (5 minutes, 4°C, 5000 rpm). The supernatant was removed and the fungal spores were inoculated with YG (Yeast Extract Glucose) medium in a volume of 20 mL. After two hours of incubation with shaking (37°C, 140 rpm), the germinated spores together with the medium were transferred to a new Falcon tube (V = 50 mL) and centrifuged (5 minutes, 4°C, 5000 rpm). After supernatant removal, the precipitate was suspended in 2 mL of sterile distilled water and transferred to a glass test tube with a stopper. According to the EUCAST standard, an alternative method was used to establish a cell density of 2 - 5 × 10⁶ CFU/mL by densitometric determination of a cell suspension density of 0.5 on the McFarland scale. The suspension prepared in this way was diluted 1:10 to obtain inoculating material of 2 - 5 × 10⁵ CFU/mL and added to the wells of the 96-well plate within 30 minutes.

Test substances: 1-methyltryptophan (1-methyl-D-tryptophan),aminooxyacetic acid (aminooxyacetic acid hemihydrochloride), 2-aminoadipic acid (DL-α-aminoadipic acid), Ro 61-8048(3,4-dimethoxy-N-[4-(3-nitrophenyl)-2-thiazolyl]-benzenesulfonamide) and KMO Inhibitor II (JM6)(2-(3,4-dimethoxybenzenesulfonylamino)-4-(3-nitrophenyl)-5-(piperidin-1-yl)methylthiazoles) were sourced from certified high-purity chemical reagent supplier, Sigma-Aldrich.

Test substance solutions were prepared in RPMI 1640 liquid medium (Gibco), containing 2% w/o glucose (Sigma-Aldrich) making the following concentrations: 0.625; 1.25; 2.5; 5.0; 10.0; 12.5; 25.0; 50.0 mM. The positive control for the test (fungal growth) was the test medium (RPMI 1640 liquid medium, containing 2% w/o glucose) and the negative control for the test (no fungal growth) was the antibiotic amphotericin B (Sigma-Aldrich), at a concentration of 4 µg/mL (4.33 mM). The experiment was conducted in 3 series of 3 repetitions. The results obtained for all three repetitions were the same. MIC values were determined based on microscopic observations of morphological changes in *Trichophyton rubrum* induced by the metabolites. In the cultures, the following were assessed: the presence of filaments and spores of the fungus. The presence of fungal hyphae in the field of view was indicative of *Trichophyton rubrum* growth and indicated absence of test compound's antifungal activity, whereas the presence of *Trichophyton* rubrum spores in the field of view indicated absence of *Trichophyton rubrum* growth and indicated an antifungal effect of the test compound.

The results of the of kynurenine pathway enzyme inhibitors' antifungal activity evaluation are shown in Table 1.

**Table 1.**

| **Substance** | **Substance Concentration [mM]** | | | | | | | | |
|---|---|---|---|---|---|---|---|---|---|
| | **0** | **0.625** | **1.25** | **2.5** | **5** | **10** | **12.5** | **25** | **50** |
| 1-Methyltryptophan | + | + | + | + | - | - | nt | nt | nt |
| Aminooxyacetic acid | + | + | - | - | - | - | nt | nt | nt |
| Aminoadipic acid | + | + | + | - | - | - | nt | nt | nt |
| Ro 61-8048 | + | + | +/- | - | - | - | - | - | - |
| KMO Inhibitor II (JM6) | + | + | + | - | - | - | - | - | - |
| Amphotericin B | - | | | | | | | | |

| | | | | | | | | | |
|---|---|---|---|---|---|---|---|---|---|
| Legend: the "+" symbol indicates the presence of *Trichophyton rubrum* filaments in the field of view and indicates fungal growth; the "+/-" symbol indicates the presence of *Trichophyton rubrum* filaments and spores in the field of view and indicates limited fungal growth. the "-" symbol indicates the presence of spores and the absence of *Trichophyton rubrum* filaments in the field of view and indicates the absence of fungal growth; "nt" - not tested. | | | | | | | | | |

### RESULTS

Among the kynurenine pathway enzyme inhibitors tested, the highest antifungal activity was found for aminooxyacetic acid, which inhibited the *Trichophyton rubrum* growth at the concentration range from 1.25 mM. Similar activity was demonstrated by Ro 61-8048, which reduced *Trichophyton rubrum* growth at a concentration of 1.25 mM and inhibited the fungal growth at concentrations of 2.5 - 50 mM. Aminoadipic acid and KMO Inhibitor II (JM6) were found to inhibit *Trichophyton rubrum* growth at concentrations from 2.5 mM and 1-methyltryptophan from a concentration of 5 mM.

### EXAMPLE 2

Compositions based on kynurenine pathway enzyme inhibitors with antifungal properties

Anhydrous nail polish compositions with the following ingredients were prepared.

| Substance | CAS No | Concentration [wt.%] | Concentration [wt.%] |
|---|---|---|---|
| | | Composition 1 | Composition 2 |
| Active substance | | 1 | 5 |
| Ethyl alcohol | 64-17-5 | 50 | 45 |
| Butyl acetate | 123-86-4 | 10 | 11 |
| Ethyl acetate | 141-78-6 | 35 | 35 |
| Triacetin | 102-76-1 | 2 | 2 |
| Ammonium methacrylate copolymer (Type A) | 33434-24-1 | 2 | 2 |

The active substance was an inhibitor of the kynurenine pathway enzyme selected from a group comprising: 1-methyltryptophan, aminooxyacetic acid, 2-aminoadipic acid, Ro 61-8048 and KMO Inhibitor II (JM6).

In order to obtain the above-mentioned compositions in a rotary mixer adapted for use with flammable and explosive solvents, a mixing process typical of obtaining therapeutic anhydrous polishes was carried out. First, a mixture of solvents (ethyl alcohol, butyl acetate, ethyl acetate, triacetin) was prepared by stirring at 300 rpm over 10 minutes, followed by the dispersion of ammonium methacrylate copolymer (type A) and the mixture was thoroughly stirred for 30 minutes at 500 rpm. Finally, the active substance was added and stirred for 10 minutes at 750 rpm.

Hydrophilic nail polish compositions with the following ingredients were prepared.

| **Substance** | **CAS No** | **Concentration [wt.%]** | **Concentration [wt.%]** |
|---|---|---|---|
| | | Composition 3 | Composition 4 |
| Active substance | n/a | 1 | 5 |
| Polyoxyl 40 hydrogenated castor oil | 61788-85-0 | 3 | 5 |
| Ethyl alcohol | 64-17-5 | 26 | 20 |
| Propylene glycol | 57-55-6 | 2 | 2 |
| Low viscosity Hydroxypropyl methylcellulose | 9004-65-3 | 1 | 1 |
| Dimethicone | 9006-65-9 | 0.5 | 0.5 |
| Hydrochloric acid | 7647-01-0 | 0.05 | 0.05 |
| Purified water | 7732-18-5 | 66.45 | 66.45 |
| final pH | | 5.5 | 3.5 |

The active substance was an inhibitor of the kynurenine pathway enzyme: 1-methyltryptophan, aminooxyacetic acid, 2-aminoadipic acid, Ro 61-8048 and KMO Inhibitor II (JM6).

To obtain the above-mentioned composition, a mixture of solvents (water, glycol and ethanol) was prepared in a rotary mixer equipped with an overhead anchor stirrer, then the active substance was added and the blend was mixed. In the next step, Polyoxyl 40 hydrogenated castor oil, low-viscosity hydroxypropyl methylcellulose, dimethicone were added successively, and the blend was mixed thoroughly. Finally, hydrochloric acid (in the form of a 28-36% w/w solution) was added until the desired pH was reached.

### BIBLIOGRAPHY

**[1]** Maleszka R, Adamski Z, Szepietowski J, Baran E. Treatment of superficial fungal infections-recommendations of experts of Mycological Section of Polish Dermatological Society. Dermatology Review/Przeglad Dermatologiczny. 2015;102(4):305-15 doi: 10.5114/dr.2015.53418;
**[2]** Martinez-Rossi NM, Bitencourt TA, Peres NTA, Lang EAS, Gomes EV, Quaresemin NR, Martins MP, Lopes L, Rossi A. Dermatophyte Resistance to Antifungal Drugs: Mechanisms and Prospectus. Front Microbiol. 2018;9:1108. doi: 10.3389/fmicb.2018.01108;
[3] see [1]
**[4]** Wang et al., Analysis of the dermatophyte Trichophyton rubrum expressed sequence tags. BMC Genomics 2006, 7:255 doi:10.1186/1471-2164-7-255;
[5] https://encyclopedia.pub/entry/27119;
**[6]** Mithaiwala MN, Santana-Coelho D, Porter GA, O'Connor JC. Neuroinflammation and the Kynurenine Pathway in CNS Disease: Molecular Mechanisms and Therapeutic Implications. Cells. 2021;10(6):1548. doi: 10.3390/cells10061548;
**[7]** Fatokun AA, Hunt NH, Ball HJ. Indoleamine 2,3-dioxygenase 2 (IDO2) and the kynurenine pathway: characteristics and potential roles in health and disease. Amino Acids. 2013;45(6):1319-29. doi: 10.1007/s00726-013-1602-1;
**[8]** Vacchelli E, Aranda F, Eggermont A, Sautès-Fridman C, Tartour E, Kennedy EP, Platten M, Zitvogel L, Kroemer G, Galluzzi L. Trial watch: IDO inhibitors in cancer therapy. Oncoimmunology. 2014;3(10):e957994. doi:10.4161/21624011.2014.957994;
[9] https://pubchem.ncbi.nlm.nih.gov/compound/286
**[10]** Nadvi NA, Salam NK, Park J, Akladios FN, Kapoor V, Collyer CA, Gorrell MD, Church WB. High resolution crystal structures of human kynurenine aminotransferase-I bound to PLP cofactor, and in complex with aminooxyacetate. Protein Sci. 2017;26(4):727-736. doi: 10.1002/pro.3119;
**[11]** Wu HQ, Ungerstedt U, Schwarcz R. L-alpha-aminoadipic acid as a regulator of kynurenic acid production in the hippocampus: a microdialysis study in freely moving rats. Eur J Pharmacol. 1995;281(1):55-61. doi: 10.1016/0014-2999(95)00224-9;
**[12]** Hodgkins PS, Wu HQ, Zielke HR, Schwarcz R. 2-Oxoacids regulate kynurenic acid production in the rat brain: studies in vitro and in vivo. J Neurochem. 1999;72(2):643-51. doi: 10.1046/j.1471-4159.1999;
**[13]** Jastrz 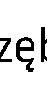 bowska K, Gabriel I. Inhibitors of amino acids biosynthesis as antifungal agents. Amino Acids. 2015;47(2):227-49. doi: 10.1007/s00726-014-1873-1;
**[14]** Greco, F. A. et al. The Janus-Faced Nature of Ido1 in Infectious Diseases: Challenges and Therapeutic Opportunities. Future Medicinal Chemistry, 2015, 8(1): 39-54;
**[15]** Han Q, Cai T, Tagle DA, Li J. Structure, expression, and function of kynurenine aminotransferases in human and rodent brains. Cell Mol Life Sci. 2010;67(3):353-68. doi: 10.1007/s00018-009-0166-4;
**[16]** Ostapiuk A, Urbanska EM. Kynurenic acid in neurodegenerative disorders-unique neuroprotection or double-edged sword? CNS Neurosci Ther. 2022;28(1):19-35. doi: 10.1111/cns.13768;
**[17]** Nematollahi A, Sun G, Jayawickrama GS, Church WB. Kynurenine Aminotransferase Isozyme Inhibitors: A Review. Int J Mol Sci. 2016;17(6):946. doi:10.3390/ijms17060946;
**[18]** Chen Y, Zhang J, Yang Y, Xiang K, Li H, Sun D, Chen L. Kynurenine-3-monooxygenase (KMO): From its biological functions to therapeutic effect in diseases progression. J Cell Physiol. 2022;237(12):4339-4355. doi: 10.1002/jcp.30876;
**[19]** Hughes TD, Güner OF, Iradukunda EC, Phillips RS, Bowen JP. The Kynurenine Pathway and Kynurenine 3-Monooxygenase Inhibitors. Molecules. 2022;27(1):273. doi: 10.3390/molecules27010273. PMID: 35011505;
**[20]** EUCAST E.DEF 9.4 Marzec 2022 r., Method for the determination of broth dilution minimum inhibitory concentrations of antifungal agents for conidia forming moulds.

## Claims

1. A kynurenine pathway enzyme inhibitor selected from the group comprising 1-methyltryptophan, aminooxyacetic acid, 2-aminoadipic acid, 3,4-dimethoxy-N-[4-(3-nitrophenyl)-2-thiazolyl]-benzenesulfonamide) and (2-(3,4-dimethoxybensenosulfonylamino)-4-(3-nitrophenyl)-5-(piperidin-1-yl)methylthiazole) alone or in combination for use in the prevention or treatment of mycoses, especially those caused by *Trichophyton rubrum.*

2. Aminooxyacetic acid for use according to claim 1, wherein its minimum effective concentration is 1.25 mM.

3. An inhibitor selected from the group consisting of 2-aminoadipic acid, 3,4-dimethoxy-N-[4-(3-nitrophenyl)-2-thiazolyl]-benzenesulfonamide) and (2-(3,4 dimethoxybensenosulfonylamino)-4-(3-nitrophenyl)-5-(piperidin-1-yl)methylthiazole) for use according to claim 1, wherein its minimum effective concentration is 2.5 mM.

4. 1-Methyltryptophan for use according to claim 1, wherein its minimum effective concentration is 5 mM.

5. Inhibitor for use according to claim 1, wherein it is administered topically, preferably in the form of nail polish.
